(19) Europäisches Patentamt
European Patent Office
Office européen des brevets

(11) EP 4 706 818 A1

(12) **EUROPEAN PATENT APPLICATION**
published in accordance with Art. 153(4) EPC

(43) Date of publication:
**11.03.2026 Bulletin 2026/11**

(21) Application number: **25745488.4**

(22) Date of filing: **21.01.2025**

(51) International Patent Classification (IPC):
***B01J 23/72*** (2006.01) ***B01J 37/00*** (2006.01)
***B01J 37/08*** (2006.01) ***B01J 21/08*** (2006.01)
***B01J 35/50*** (2024.01) ***B01J 37/03*** (2006.01)
***C07C 29/141*** (2006.01) ***C07C 31/20*** (2006.01)

(52) Cooperative Patent Classification (CPC):
**B01J 21/08; B01J 23/72; B01J 35/50; B01J 37/00; B01J 37/03; B01J 37/08; C07C 29/141; C07C 31/20**

(86) International application number:
**PCT/KR2025/001144**

(87) International publication number:
**WO 2025/159471 (31.07.2025 Gazette 2025/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.01.2024 KR 20240009953**

(71) Applicant: **LG Chem, Ltd.**
**Seoul 07336 (KR)**

(72) Inventors:
- **SONG, Cheolock**
  **Daejeon 34122 (KR)**
- **SUH, Myungji**
  **Daejeon 34122 (KR)**
- **BYEON, Jiyeon**
  **Daejeon 34122 (KR)**

(74) Representative: **Hoffmann Eitle**
**Patent- und Rechtsanwälte PartmbB**
**Arabellastraße 30**
**81925 München (DE)**

(54) **COPPER-BASED COMPOSITE CATALYST AND METHOD FOR DISTINGUISHING OXIDATION NUMBERS THEREOF**

(57) The present disclosure relates to a copper-based composite catalyst and a method for discriminating the same, characterized in that it has a specific content of copper on its surface and a specific ratio depending on the oxidation number of copper. The copper-based composite catalyst according to the present disclosure has almost no change in strength and has excellent reactivity when used in the preparation of neopentyl glycol (NPG).

【FIG. 1】

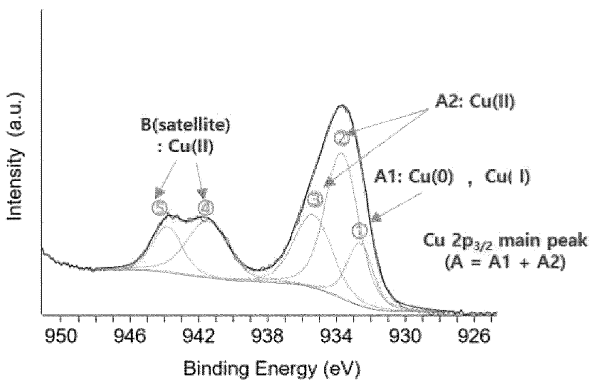




Processed by Luminess, 75001 PARIS (FR)

## Description

[Technical Field]

The present disclosure relates to a copper-based composite catalyst and a method for discriminating the oxidation number thereof.

<Cross-reference to related applications>

This application claims the benefit of Korean Patent Application No. 10-2024-0009953 filed in the Korean Intellectual Property Office on January 23, 2024, the entire contents of which are incorporated herein by reference.

[Background Art]

Neopentyl glycol (NPG) is a white crystalline substance with a melting point of 130 °C or higher. It is used as an important intermediate for various synthetic resins and is also widely used industrially as a raw material for various plastic powder paints, synthetic lubricants, plasticizers, surfactants, fiber processing agents, etc.

The NPG is generally prepared by preparing hydroxypivaldehyde (HPA) through an aldol condensation reaction of isobutyraldehyde and formaldehyde, and then reacting the HPA with hydrogen in the presence of a catalyst. At this time, the process is carried out under high-temperature (160 °C or higher) and high-pressure (35 bar or higher) conditions using a copper-based composite catalyst to achieve a high yield.

However, when the copper-based composite catalyst is used, because the reaction proceeds under high-temperature and high-pressure conditions, the strength of the catalyst is an important factor, and accordingly, a copper-based composite catalyst containing copper oxide (CuO) is used in the art.

Since the copper-based composite catalyst exhibits large difference in reaction activity depending on the oxidation number of copper, it is important to determine not only the content of copper constituting the catalyst but also the component ratio of copper with different oxidation numbers in order to control the activity of the catalyst. It is important to accurately measure the ratio of copper with different oxidation numbers in the copper-based composite catalyst in order to control the composition ratio of copper with a specific oxidation number and to confirm the preparation of a catalyst with desired physical properties.

Therefore, in order to improve the physical properties of a catalyst, such as the stability and activity, the development of a technology for accurately measuring and determining the composition ratio of copper having a specific oxidation number is being carried out continuously.

## [Disclosure]

[Technical Problem]

The present specification provides a copper-based composite catalyst and a method for discriminating the oxidation number thereof.

[Technical Solution]

An exemplary embodiment of the present specification provides a copper-based composite catalyst containing copper at a content of 2.5 at% to 7.5 at% on the surface, wherein the copper includes copper with an oxidation number of 0 and copper with an oxidation number of 1 at a ratio of 10% to 50% and copper with an oxidation number of 2 at a ratio of 50% to 90%, and the content and ratio are determined by the following method 1.

[Method 1]

An XPS spectrum is obtained for the surface of the copper-based composite catalyst, which includes a main peak for copper with an oxidation number of 0, a main peak for copper with an oxidation number of 1, a main peak for copper with an oxidation number of 2, and satellite peaks for copper, and

the total content (at%) of copper and the ratio (%) of copper with oxidation numbers of 0 and 1 and copper with an oxidation number of 2 are determined using the area of the XPS spectrum.

Another exemplary embodiment of the present specification provides a method for discriminating the oxidation number of a copper-based composite catalyst, which includes: a step of obtaining an XPS spectrum for the surface of a copper-based composite catalyst, which includes a main peak for copper with an oxidation number of 0, a main peak for

copper with an oxidation number of 1, a main peak for copper with an oxidation number of 2, and satellite peaks for copper; and a step of calculating the total content (at%) of copper and the ratio (%) of copper with oxidation numbers of 0 and 1 and copper with an oxidation number of 2 using the area of the XPS spectrum.

[Advantageous Effects]

A copper-based composite catalyst according to the present disclosure has high strength, exhibits almost no change in strength before and after post-treatment, exhibits almost no copper elution during storage, treatment or use, exhibits very fast hydrogen consumption reaction rate when used in the preparation of neopentyl glycol (NPG), and has excellent reactivity.

A method for discriminating the oxidation number of a copper-based composite catalyst according to the present disclosure allows accurate measurement the content of copper with a specific oxidation number in the copper-based composite catalyst, and also allows calculation of the content of copper with a specific oxidation number, existing in a trace amount.

[Brief Description of Drawings]

FIGS. 1 to 3 are XPS spectra of copper-based composite catalysts (Examples 1-1 to 1-3 in sequence) prepared according to a method for discrimination according to an exemplary embodiment of the present specification.

FIG. 4 and FIG. 5 are XPS spectra of copper-based composite catalysts (Comparative Examples 1-1 and 1-2 in sequence) for comparison with the exemplary embodiments of the present specification.

FIGS. 6 to 9 are XPS spectra of a copper-based composite catalyst according to an exemplary embodiment of the present specification (Example 2-3 (FIG. 8)) and copper-based composite catalysts for comparison with the exemplary embodiment of the present specification (Comparative Examples 2-2 (FIG. 6), 2-3 (FIG. 7) and 2-4 (FIG. 9).

[Best Mode]

Hereinafter, the present disclosure will be described in detail so that a person with ordinary skill in the art can easily carry out the present disclosure. However, the present disclosure can be embodied in various different forms and is not limited to the configuration described herein.

In this specification, when a part is said to 'include' a certain component, this means that it may include other components, but not excludes other components, unless the context specifically states otherwise.

In this specification, 'p to q' means 'p or more and q or less'.

In this specification, Cu(0) represents copper with an oxidation number of 0, Cu(I) represents copper with an oxidation number of 1, and Cu (II) represents copper with an oxidation number of 2.

In this specification, the Cu $2p_{3/2}$ main peak refers to a sharp peak whose intensity increases rapidly within the binding energy (B.E.) range of 928.0 eV to 939.0 eV in the 2p XPS spectrum of Cu, and the satellite peak refers to a peak whose intensity increases gradually within the binding energy (B.E.) range of 939.0 eV to 950.0 eV (see FIGS. 1 to 9).

In this specification, the main peak for copper with an oxidation number of 0, the main peak for copper with an oxidation number of 1, and the main peak for copper with an oxidation number of 2 can be expressed as a Cu(0) main peak, a Cu(I) main peak, and a Cu(II) main peak, respectively.

In this specification, "strength" means the detachment strength of a catalyst. Specifically, it may mean a value calculated from the average of 20 maximum detachment strengths at the initial collapse site using SHIMPO's FGN-50B.

And, in describing the present disclosure, detailed descriptions of related known technologies that may unnecessarily obscure the gist of the present disclosure will be omitted.

**<Copper-based composite catalyst>**

An exemplary embodiment of the present specification provides a copper-based composite catalyst containing copper at a content of 2.5 at% to 7.5 at% on the surface, wherein the copper includes copper with an oxidation number of 0 and copper with an oxidation number of 1 at a ratio of 10% to 50% and copper with an oxidation number of 2 at a ratio of 50% to 90%, and the content and ratio are determined by the following method 1.

[Method 1]

An XPS spectrum is obtained for the surface of the copper-based composite catalyst, which includes a main peak for copper with an oxidation number of 0, a main peak for copper with an oxidation number of 1, a main peak for copper with an oxidation number of 2, and satellite peaks for copper, and
the total content (at%) of copper and the ratio (%) of copper with oxidation numbers of 0 and 1 and copper with an oxidation number of 2 are determined using the area of the XPS spectrum.

The copper-based composite catalyst satisfying the above-described content (at%) and ratio (%) exhibits high strength, improved stability with little change in strength before and after post-treatment, and very fast hydrogen consumption reaction rate and excellent reactivity when used in the preparation of neopentyl glycol (NPG).

In this specification, the content (at%) can be expressed as an atomic concentration.

In this specification, the main peaks for different oxidation numbers and satellite peaks are calculated based on the area of the XPS spectrum. The oxidation number can be determined and/or quantified using the area for each peak according to the method described specifically in this specification.

In an exemplary embodiment of the present specification, the ratio (%) of copper with oxidation numbers of 0 and 1 can be calculated from Equation 1, and the ratio (%) of copper with an oxidation number of 2 can be calculated from Equation 2.

[Equation 1]

$$\%\,(\mathrm{Cu(0)} + \mathrm{Cu(I)}) = \frac{(\mathrm{A1} + \mathrm{A2}) - (\mathrm{As/Bs})\mathrm{B}}{(\mathrm{A1} + \mathrm{A2}) + \mathrm{B}} \times 100(\%)$$

[Equation 2]

$$\%\,\mathrm{Cu(II)} = \frac{\mathrm{B}(1 + (\mathrm{As/Bs}))}{(\mathrm{A1} + \mathrm{A2}) + \mathrm{B}} \times 100(\%)$$

In Equations 1 and 2,
Cu(0), Cu(I), and Cu(II) represent copper with an oxidation number of 0, copper with an oxidation number of 1, and copper with an oxidation number of 2, respectively, and satisfy the following relationship:

$$\%\,(\mathrm{Cu(0)} + \mathrm{Cu(I)}) + \%\,\mathrm{Cu(II)} = 100(\%)$$

,

A1 is the sum of the total area of the Cu(0) main peak and the total area of the Cu(I) main peak calculated from the XPS spectrum,
A2 is the total area of the Cu(II) main peak calculated from the XPS spectrum,
B is the total area of satellite peaks for Cu(II) calculated from the XPS spectrum, and
As/Bs is the area ratio of main peak/satellite peaks for Cu $2p_{3/2}$ of Cu(II).

In this specification, the ratio of copper of the copper-based composite catalyst depending on oxidation numbers is the value obtained by Equations 1 and 2, respectively. Specifically, the ratio (%) of copper with oxidation numbers of 0 and 1 can be obtained by calculation according to Equation 1, and the ratio (%) of copper with an oxidation number of 2 can be obtained by calculation according to Equation 2.

The copper-based composite catalyst according to an exemplary embodiment of the present specification may be treated further by a method known in the art without departing from the scope of the present disclosure. The additional treatment may include hydrothermal treatment, etc.

In this specification, hydrothermal treatment is one of post-treatments for controlling the strength of the catalyst, etc. The hydrothermal treatment method is not particularly limited as long as it is a method known in the art and does not deviate from the present disclosure.

In an exemplary embodiment of the present specification, the surface may mean a region having a depth of 0 nm or larger and 10 nm or smaller in a center direction from the interface of the copper-based composite catalyst in contact with the atmosphere.

In an exemplary embodiment of the present specification, the area of the XPS spectrum may mean the total area of the main peak for copper with an oxidation number of 0 and the main peak for copper with an oxidation number of 1 (first area value) in the XPS spectrum, the total area of the main peak for copper with an oxidation number of 2 (second area value), and the total area of the satellite peaks for copper (third area value).

In an exemplary embodiment of the present specification, the first to third area values may be measured through fitting treatment using the Lorentzian/Gaussian (L/G) function and the FWHM (full width at half maximum) after setting the background using Shirley's method.

In an exemplary embodiment of the present specification, the copper-based composite catalyst may contain at least one of a copper oxide and a copper structure.

In this specification, the copper oxide can be expressed as Cu-O, and the copper structure can be expressed as

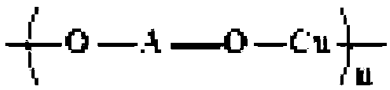

(n is an integer greater than or equal to 1, with the upper limit undefined, and A is an element such as Al, Si, etc.).

In this specification, the copper oxide and the copper structure are catalyst structures containing copper, and mean materials having catalytic activity and playing a main role in catalytic reactions.

In an exemplary embodiment of the present specification, the copper-based composite catalyst may further contain a support.

In this specification, the support is a material that disperses and maintains the copper oxide and structure on its surface. Representative examples include silica and alumina. But zeolite, titania, magnesia, zirconia, carbon, diatomaceous earth, etc. can also be used as the support. Any method known in the art may be used without particular limitation as long as it does not deviate from the present disclosure.

The copper-based composite catalyst according to an exemplary embodiment of the present specification may be a catalyst for preparing neopentyl glycol. Accordingly, when the copper-based composite catalyst is used in the preparation of neopentyl glycol, reactivity such as reaction efficiency is improved, and the stability of the catalyst structure itself can be improved without change in strength.

**<Method for discriminating oxidation number of copper-based composite catalyst>**

An exemplary embodiment of the present specification provides a method for discriminating the oxidation number of a copper-based composite catalyst using X-ray photoelectron spectroscopy (XPS).

More specifically, the method for discriminating the oxidation number of a copper-based composite catalyst according to the present specification is characterized in that the total content (at%) of copper and the ratio of copper depending on the oxidation number are calculated by using the area of the XPS spectrum for the copper-based composite catalyst, which includes a main peak for copper with an oxidation number of 0, a main peak for copper with an oxidation number of 1, a main peak for copper with an oxidation number of 2, and satellite peaks for copper.

The method for discriminating the oxidation number of a copper-based composite catalyst according to an exemplary embodiment of the present specification is applicable to a copper-based composite catalyst that contains copper at a content of 0.1 at% or higher on the surface.

Within the above range, the method for discriminating the oxidation number of a copper-based composite catalyst according to the present disclosure can be applied.

Due to the detection limit of XPS, the method for discriminating the oxidation number of a copper-based composite catalyst according to the present disclosure cannot be applied when the total content of copper with an oxidation number of 0 and copper with an oxidation number of 1 is below 0.05 at%. Also, when the total content of copper with an oxidation number of 0 and copper with an oxidation number of 1 is 100 at%, there is no reason to discriminate the content of copper depending on oxidation number since it is copper metal or $Cu_2O$.

The copper-based composite catalyst according to an exemplary embodiment of the present specification is not limited specially, except that it satisfies the above-described contents of copper with an oxidation number of 0 [Cu(0)], copper with an oxidation number of 1 [Cu(I)], and copper with an oxidation number of 2 [Cu(II)], as measured by the method for discriminating the oxidation number of a copper-based composite catalyst.

In the method for discriminating the oxidation number of a copper-based composite catalyst according to the present specification, the ratio (%) of copper with oxidation numbers of 0 and 1 can be calculated from Equation 1, and the ratio (%) of copper with an oxidation number of 2 can be calculated from Equation 2. The foregoing description of the

copper-based composite catalyst can be equally applied to Equations 1 and 2.

The method for discriminating the oxidation number of a copper-based composite catalyst according to an exemplary embodiment of the present specification can determine the content of a specific oxidation number more accurately by utilizing not only the main peaks but also the satellite peaks. In addition, there is an advantage in that the content of copper for a specific desired oxidation number in a copper-based composite catalyst can be accurately determined even in a trace range.

In an exemplary embodiment of the present specification, the step of obtaining the XPS spectrum of the copper-based composite catalyst may include: a step of obtaining the XPS spectrum of the copper-based composite catalyst by X-ray photoelectron spectroscopy (XPS) under conditions of a vacuum atmosphere, a measurement range of 925 eV to 970 eV, and a pass energy of 50 eV; and a step of correcting the peaks of the XPS spectrum.

In this specification, the XPS spectrum of the copper-based composite catalyst may be obtained under the conditions of a vacuum atmosphere, a measurement range of 925 eV to 970 eV, and a pass energy of 50 eV.

That is, after a first XPS spectrum (XPS spectrum before correction) of the copper-based composite catalyst is obtained by X-ray photoelectron spectroscopy (XPS) under the conditions of a vacuum atmosphere, a measurement range of 925 eV to 970 eV, and a pass energy of 50 eV, a second XPS spectrum (XPS spectrum after correction) which is the analysis target of the present disclosure can be obtained by correcting the peaks of the XPS spectrum.

In an exemplary embodiment of the present specification, the copper-based composite catalyst may be prepared into a circular pellet sample having a diameter of 2 mm and a thickness of 1 mm, and an XPS spectrum may be obtained from the sample using Al k-alpha X-ray with a size of 400 μm x 800 μm under the XPS obtainment conditions described above. That is, monochromatic Al Kα (1486.6 eV) can be used as an X-ray source for XPS spectrum analysis, and Shirley peak background, ALTHERMO1 sensitivity factor, and TPP-2M energy compensation factor can be applied. Additionally, the contents of elements present on the surface of the copper-based composite catalyst (atomic percent, at%, based on the total atomic weight of the elements present on the surface) can be calculated using the Avantage software.

In an exemplary embodiment of the present specification, in the step of correcting the peaks of the XPS spectrum, the C-C binding energy for graphite of the C 1s spectrum may be fixed to an energy of a certain size, and the binding energy for the main peak for copper may be corrected based on the energy of the certain size. The energy of the certain size may be 284 eV to 285 eV, and most specifically 284.8 eV.

In an exemplary embodiment of the present specification, the correction based on the energy of a certain size may mean the correction of the binding energy of the Cu(I) main peak.

Through the above process, the range of peak binding energy for obtaining the Cu(II) peak area can be set to 933.3 eV to 936.0 eV, and the range of peak binding energy for obtaining the peak areas of Cu(0) and Cu(I) can be set to 932.0 eV to 932.8 eV.

When the ranges of peak binding energy are set as described above, pure peaks can be found. Since the content of the copper-based composite catalyst depending on the oxidation number can be determined by finding pure peaks not affected by other factors, the accuracy of the analysis can be improved.

In an exemplary embodiment of the present specification, the step of calculating the total content (at%) of copper and the ratio (%) of copper with oxidation numbers of 0 and 1 and copper with an oxidation number of 2 using the area of the XPS spectrum may include:

a step of obtaining the total content of copper on the surface of the copper-based composite catalyst from the XPS spectrum;
a step of obtaining a first area value as the total area for the main peak for copper with an oxidation number of 0 and the main peak for copper with an oxidation number of 1 on the surface of the copper-based composite catalyst from the XPS spectrum;
a step of obtaining a second area value as the total area of the main peak for copper with an oxidation number of 2 on the surface of the copper-based composite catalyst from the XPS spectrum;
a step of obtaining a third area value as the total area of the satellite peaks for copper on the surface of the copper-based composite catalyst from the XPS spectrum; and
a step of obtaining the content (at%) of copper with oxidation numbers of 0 and 1 and the content (at%) of copper with an oxidation number of 2 from Equations 4 and 5.

[Equation 4]

$$\text{at\%}(\text{Cu}(0) + \text{Cu}(\text{I})) = \text{at\%}(\text{Cu}) \times \frac{\%(\text{Cu}(0) + \text{Cu}(\text{I}))}{100(\%)}$$

[Equation 5]

$$\text{at\% Cu(II)} = \text{at\%(Cu)} \times \frac{\text{\% Cu(II)}}{100(\%)}$$

In Equations 4 and 5,

at%(Cu(0) + Cu(I)) is the total content of copper with an oxidation number of 0 and copper with an oxidation number of 1 in the copper-based composite catalyst,
at% Cu(II) is the total content of copper with an oxidation number of 2 in the copper-based composite catalyst,
% (Cu(0) + Cu(I)) is the ratio (%) of copper with oxidation numbers of 0 and 1 calculated from Equation 1, and
% Cu(II) is the ratio (%) of copper with an oxidation number of 2 calculated from Equation 2.

In this specification, the peaks of Cu(0) and Cu(I) can be expressed as Cu (0) + Cu (I) peaks. For reference, the Cu (0) + Cu (I) peak may refer to the main peaks for Cu(0) and Cu(I).

In an exemplary embodiment of the present specification, the step of obtaining the first area value may include: a step of finding both a main peak for copper with an oxidation number of 0 and a main peak for copper with an oxidation number of 1 from the XPS spectrum; and a step of calculating the area values of the main peak for copper with an oxidation number of 0 and the main peak for copper with an oxidation number of 1, and then summing the calculated area values.

In the case of the Cu (0) main peak and Cu (I) main peak, since more than one peak may appear in the XPS spectrum, both the Cu (0) main peak and the Cu(I) main peak must be found for accurate measurement. In addition, since it may be difficult to distinguish the Cu (0) main peak and Cu (I) main peak in the spectrum, the total area value of the Cu (0) main peak and Cu (I) main peak is obtained. Referring to FIG. 1, there exists one Cu(0) main peak or Cu (I) main peak (see ① in FIG. 1), but this is only an example and there may exist multiple Cu (0) main peaks or Cu (I) main peaks. The first area value corresponds to the area value of A1 in FIG. 1.

In an exemplary embodiment of the present specification, the step of obtaining the second area value may include: a step of finding all main peaks for copper with an oxidation number of 2 from the XPS spectrum; and a step of calculating the area value of each main peak for copper with an oxidation number of 2, and then summing the calculated area values. Since one or more Cu(II) main peak can appear in the XPS spectrum, accurate measurement is possible only when all the Cu(II) main peaks are found. That is, as can be seen from FIG. 1, since there can be multiple Cu(II) main peaks (see ② and ③ in FIG. 1), accurate measurement is possible only when all the Cu(II) main peaks in the XPS spectrum are found. The second area value corresponds to the area value of A2 in FIG. 1.

In an exemplary embodiment of the present specification, the step of obtaining the third area value may include: a step of finding all satellite peaks for copper from the XPS spectrum; and a step of calculating the area values of each satellite peak for copper, and then summing the calculated area values. Since one or more satellite peaks can appear in the XPS spectrum, accurate measurement is possible only when all the satellite peaks are found. That is, as can be seen from FIG. 1, since there can be multiple Cu(II) satellite peaks (see ④ and ⑤ in FIG. 1), accurate measurement is possible only when all the satellite peaks in the XPS spectrum are found. The third area value corresponds to the area value of B in FIG. 1.

In an exemplary embodiment of the present specification, the satellite peaks may be satellite peaks for copper with an oxidation number of 2. They can be expressed as Cu(II) satellite peaks. That is, the Cu (II) satellite peaks may mean satellite peaks for copper with an oxidation number of 2 found in the XPS spectrum after the correction (second spectrum).

The method for discriminating the oxidation number of a copper-based composite catalyst according to an exemplary embodiment of the present specification is characterized in that not only the main peaks but also the satellite peaks are utilized. Therefore, clearly finding the satellite peaks is a very important part in increasing the accuracy of the measurement results.

In an exemplary embodiment of the present specification, As/Bs may be 1.5 to 2.1.

In an exemplary embodiment of the present specification, the copper-based composite catalyst may contain at least one of a copper oxide and a copper structure, and may further contain a support in some cases. The foregoing description of the copper-based composite catalyst may also apply here.

For example, the copper-based composite catalyst may include a Cu-O-Si catalyst, a Cu-O-Al catalyst, a Cu-O-Si-Al catalyst, etc.

The Cu-O-Si catalyst is a catalyst containing a copper oxide (Cu-O) and a support (silica), and examples thereof include $CuO/SiO_2$, $CuO/BaO/SiO_2$, $CuO/ZnO/SiO_2$, $CuO/BaO/SiO_2$, $CuO/ZnO/SiO_2$, $CuO/MnO/SiO_2$, $CuO/Cr_2O_3/SiO_2$, copper phyllosilicate (...O-Si-O-Cu-O-Si-O-Cu-O...) as a copper-silicate structure catalyst, etc.

The Cu-O-Al catalyst is a catalyst containing a copper oxide and a support (alumina), and examples thereof include $CuO/Al_2O_3$, copper aluminate (...O-Al-O-Cu-O-Al-O-Cu-O...) as a copper-aluminate structure catalyst, etc.

The Cu-O-Si-Al catalyst is a catalyst containing a copper oxide and a support (alumina or silica) and may be, for example, $CuO/Al_2O_3/SiO_2$.

When the copper-based composite catalyst further contains a support, it can be selected from a $CuO/SiO_2$ catalyst, a $CuO/Al_2O_3$ catalyst, a $CuO_x/SiO_y$ catalyst ($0 \leq x < 1$, $0 \leq y < 2$), and a $CuO_x/AlO_y$ catalyst ($0 \leq x < 1$, $0 \leq y < 1.5$).

The examples of the copper-based composite catalysts are not limited to those described above.

The copper-based composite catalyst prepared according to the present disclosure may be a catalyst for preparing neopentyl glycol. In this case, in an exemplary embodiment of the present specification, the neopentyl glycol may be prepared by utilizing a method known in the art, except for using the copper-based composite catalyst according to the present disclosure.

More specifically, an exemplary embodiment of the present specification provides a method for preparing neopentyl glycol, which includes a step of introducing a hydroxypivaldehyde (HPA) solution and hydrogen into a hydrogenation reactor to perform a hydrogenation reaction, wherein the hydrogenation reactor contains the copper-based composite catalyst according to the present disclosure.

For example, the hydrogenation reactor may be a fixed bed reactor (FBR) filled with the copper-based composite catalyst (catalyst for preparing neopentyl glycol), in which case there is no need for separation of the catalyst and the reaction product, the reaction temperature and reaction pressure can be lowered as compared to the existing method, so that operation is stable and economical, and the exchange of the catalyst is easy, and the reactor size can be reduced. Therefore, the investment cost can be reduced significantly.

In an exemplary embodiment of the present specification, the hydroxypivaldehyde solution may contain 50 to 80 wt% of hydroxypivaldehyde, 1 to 5 wt% of neopentyl glycol, 15 to 35 wt% of an alcohol, and 1 to 10 wt% of water, in which case the heat of reaction can be minimized without lowering reactivity. Therefore, the production of byproducts can be suppressed.

In addition, in an exemplary embodiment of the present specification, the hydrogenation reaction may be carried out at a reaction temperature of 100 °C to 250 °C, specifically 100 °C to 200 °C, and more specifically 100 °C to 180 °C.

In addition, in an exemplary embodiment of the present specification, the hydrogenation reaction may be carried out at a reaction pressure of 35 bar or higher. The reaction pressure refers to the measured pressure.

When the catalyst for preparing neopentyl glycol according to an exemplary embodiment of the present specification is applied to the preparation of neopentyl glycol, the problem of elution of catalyst components (e.g., copper components) into the prepared neopentyl glycol solution can be prevented.

Therefore, when the catalyst for preparing neopentyl glycol according to an exemplary embodiment of the present specification is applied to the preparation of neopentyl glycol, the preparation cost can be reduced since a purification process due to the elution catalyst components (e.g., copper components) is not required, and the lifespan of the catalyst is extended.

[Mode for Invention]

Hereinafter, the present disclosure will be described in detail through examples in order to explain the present disclosure specifically. However, the examples according to the present disclosure may be modified in various different forms, and the scope of the present disclosure is not construed as being limited to the examples described below. The examples of the present disclosure are provided to more fully describe the present disclosure to those of ordinary skill in the art.

### Test Example 1. Evaluation of method for discriminating oxidation number of copper-based composite catalyst

#### <Example 1-1>

A copper-based composite Cu-O-Si catalyst was prepared by the following process as described in Table 1. A precipitated powder was obtained through co-precipitation of Cu nitrate, silica sol, and an alkaline precipitant. The Cu/Si wt% ratio was 30:70 = 0.43.

Afterwards, dried powder was obtained through filtration, rinsing and drying.

To analyze the Cu oxidation number ratio on the catalyst surface, the copper-based composite Cu-O-Si catalyst sample was prepared into a circular pellet with a diameter of 2 mm and a thickness of 1 mm through a drying/calcination process.

The sample was placed in an XPS (X-ray photoelectron spectroscopy) instrument (model name/manufacturer: Nexsa/ThermoScientific), and the Cu 2p spectrum was obtained by measuring using Al k-alpha x-ray with a size of 400 $\mu$m x 800 $\mu$m at 1486.6 eV in a vacuum atmosphere (~$10^{-7}$ torr), with a measurement range of 925 eV to 970 eV, and a pass energy of 50 eV. Next, the binding energy of the obtained spectrum was corrected to match the graphite C-C binding

energy of 284.8 eV of the carbon 1s (C 1s) spectrum to obtain the XPS spectrum shown in FIG. 1.

In the XPS spectrum, the background was set using the Shirley method, and the first area value (A1) of the Cu(0) main peak, which represents copper with an oxidation number of 0, or the Cu(I) main peak, which represents copper with an oxidation number of 1, the second area value (A2), which is the total area of the Cu(II) main peak representing copper with an oxidation number of 2, and the third area value (B) of the Cu(II) satellite peaks, which represents the satellite peaks for copper with an oxidation number of 2, were calculated through fitting using the Lorentzian/Gaussian (L/G) function and the full width at half maximum (FWHM).

Specifically, the Lorentzian/Gaussian (L/G) function ratio was set to 70/30, and the FWHM fit parameter was set to 0.5:3.5.

The obtainment of the XPS spectrum, background setting, and fitting treatment were performed using the Avantage software.

Next, after obtaining the first to third area values, the ratio of copper with an oxidation number of 2 of the copper-based composite catalyst of Example 1-1 was calculated using Equation 2. And, the ratio of copper with an oxidation number of 0 and copper with an oxidation number of 1 was calculated using Equation 1.

The symbols in Equations 1 and 2 have the meanings described below and, for As/Bs, the known CuO average value of 1.89 was used because the sample of Example 1-1 is a Cu-O-Si catalyst and contains a Cu-O bond.

[Equation 1]

$$\%\,(\mathrm{Cu(0)} + \mathrm{Cu(I)}) = \frac{(\mathrm{A1} + \mathrm{A2}) - (\mathrm{As/Bs})\mathrm{B}}{(\mathrm{A1} + \mathrm{A2}) + \mathrm{B}} \times 100(\%)$$

[Equation 2]

$$\%\,\mathrm{Cu(II)} = \frac{\mathrm{B}(1 + (\mathrm{As/Bs}))}{(\mathrm{A1} + \mathrm{A2}) + \mathrm{B}} \times 100(\%)$$

In Equations 1 and 2,

Cu(0), Cu(I), and Cu(II) represent copper with an oxidation number of 0, copper with an oxidation number of 1, and copper with an oxidation number of 2, respectively, and satisfy the following relationship:

$$\%\,(\mathrm{Cu(0)} + \mathrm{Cu(I)}) + \%\,\mathrm{Cu(II)} = 100(\%)$$

,

A1 is the sum of the total area of the Cu(0) main peak and the total area of the Cu(I) main peak calculated from the XPS spectrum,
A2 is the total area of the Cu(II) main peak calculated from the XPS spectrum,
B is the total area of satellite peaks for Cu(II) calculated from the XPS spectrum, and
As/Bs is the area ratio of main peak/satellite peaks for Cu $2p_{3/2}$ of Cu(II).

The content of copper (at%) measured by XPS and the ratio values depending on the oxidation number of copper calculated from Equations 1 and 2 were used to calculate the total content of copper with an oxidation number of 0 and copper with an oxidation number of 1 and the total content of copper with an oxidation number of 2 on the surface of the copper-based composite catalyst of Example 1-1, using Equations 4 and 5. The meanings of the symbols in Equations 4 and 5 are described below, and the resulting values are listed in Table 1.

[Equation 4]

$$at\%(Cu(0) + Cu(I)) = at\%(Cu) \times \frac{\%(Cu(0) + Cu(I))}{100(\%)}$$

[Equation 5]

$$at\%\ Cu(II) = at\%(Cu) \times \frac{\%\ Cu(II)}{100(\%)}$$

In Equations 4 and 5,

at%(Cu(0) + Cu(I)) is the total content of copper with an oxidation number of 0 and copper with an oxidation number of 1 in the copper-based composite catalyst,
at% Cu(II) is the total content of copper with an oxidation number of 2 in the copper-based composite catalyst,
% (Cu(0) + Cu(I)) is the ratio (%) of copper with oxidation numbers of 0 and 1 calculated from Equation 1, and
% Cu(II) is the ratio (%) of copper with an oxidation number of 2 calculated from Equation 2.

**<Examples 1-2 and 1-3>**

The Cu/Si wt% ratio was 30:70 = 0.43, and the total content of copper with an oxidation number of 0 and copper with an oxidation number of 1 and the total content of copper with an oxidation number of 2 on the surface of the copper-based composite catalysts of Examples 1-2 and 1-3 were calculated in the same manner as in Example 1-1.
The resulting values are shown in Table 1.
Additionally, the XPS spectra of Examples 1-2 and 1-3 are as shown in FIGS. 2 and 3, respectively.

**<Comparative Example 1-1>**

An XPS spectrum was obtained using the same copper-based composite catalyst as in Example 1-1 using the same method as in Example 1-1. However, unlike Example 1-1, background setting and fitting treatment were performed without considering the satellite peaks.
As a result, as shown in FIG. 4, the area (A1) of the Cu(0) and Cu (I) main peaks representing copper with an oxidation number of 0 or 1 on the surface and the area (B) of the satellite peaks were absent, and only the total area (A2) of the Cu(II) main peak representing copper with an oxidation number of 2 was observed. That is, in the case of Comparative Example 1-1, even though the same catalyst as in Example 1-1 was used, the ratio of copper with an oxidation number of 0 and copper with an oxidation number of 1 could not be measured.
Next, the ratio of copper with an oxidation number of 2 was measured from the fitting result shown in FIG. 4.
The result is shown in Table 1.

**<Comparative Example 1-2>**

The content and oxidation number of copper in the copper-based composite catalyst were measured in the same manner as in Comparative Example 1-1, except that the same copper-based composite catalyst as in Example 1-2 was used.
That is, as shown in FIG. 5, since there was no area (A1) of the Cu (0) and Cu (I) main peaks representing copper with an oxidation number of 0 or 1, and no area (B) of the satellite peaks, the ratio of copper with an oxidation number of 0 and copper with an oxidation number of 1 could not be measured also in Comparative Example 1-2. The ratio of copper with an oxidation number of 2 was measured from the fitting result shown in FIG. 5. The result is shown in Table 1.

[Table 1]

| Ratio (%) depending on oxidation number of Cu / content (at %) depending on oxidation number of Cu | Cu-O-Si catalyst | | | | |
|---|---|---|---|---|---|
| | Ex. 1-1 | Ex. 1-2 | Ex. 1-3 | Comp. Ex. 1-1 | Comp. Ex. 1-2 |
| Cu(0), Cu(I) | 18 / 0.67 | 7 / 0.57 | 27 / 1.0 | 0 / 0.0 | 0 / 0.0 |
| Cu (II) | 82 / 3.03 | 93 / 7.53 | 73 / 2.8 | 100 / 3.7 | 100 / 8.1 |

From the result shown in Table 1, it was confirmed that the content of copper depending on oxidation number can be determined more accurately by the method for discrimination according to the present disclosure (Examples 1-1 to 1-3) than when the method for discrimination according to the present disclosure was not used (Comparative Examples 1-1 and 1-2).

That is, when the satellite peaks were not considered, because the area (A1) of the Cu (0) and Cu (I) main peaks representing copper with an oxidation number of 0 or 1 did not exist, the content of copper with an oxidation number of 0 or 1 could not be determined. Additionally, the content of copper with an oxidation number of 2 was also measured inaccurately because the content of copper with an oxidation number of 0 or 1 could not be determined.

**Test Example 2. Evaluation of physical properties of copper-based composite catalyst**

**<Examples 2-1 and 2-2>**

The physical properties of the Cu-O-Si catalysts of Examples 1-1 and 1-3 were evaluated.

**<Example 2-3>**

A Cu-O-Al catalyst prepared by applying the method for discrimination described in Example 1-1 was used as a copper-based composite catalyst for evaluation of physical properties.

In addition, a copper-based composite catalyst was prepared in the same manner as in Example 1-1 through filtration, rinsing, drying processes, preparation into circular pellets, and drying/calcination, except that Cu nitrate, alumina sol, and an alkaline precipitant were added so that at the Cu/Al wt% ratio was 30:70 to prepare a precipitate powder by co-precipitation.

**<Comparative Example 2-1>**

The Cu-O-Si catalyst of Example 1-2 was used as a copper-based composite catalyst for evaluation of physical properties.

**<Comparative Example 2-2>**

A Cu-O-Al catalyst was prepared in the same manner as described in Example 2-3, except that the precipitate powder was prepared by adding Cu/Al at a wt% ratio of 15:85.

**<Comparative Example 2-3>**

A Cu-O-Al catalyst was prepared in the same manner as described in Example 2-3, except that the precipitate powder was prepared by adding Cu/Al at a wt% ratio of 15:85 and hydrothermal treatment was performed at 150 °C to increase the ratio (%) of Cu(II).

**<Comparative Example 2-4>**

A Cu-O-Al catalyst was prepared in the same manner as described in Example 2-3, except that hydrothermal treatment was performed at 150 °C to increase the ratio (%) of Cu(II).

As described in Table 2, the contents of copper with an oxidation number of 0, copper with an oxidation number of 1, and copper with an oxidation number of 2 in the copper-based composite Cu-O-Al catalyst were calculated in the same manner as in Example 1-1, and the resulting values are as follows. Additionally, the XPS spectra for Comparative Examples 2-2 and 2-3, Example 2-3, and Comparative Example 2-4 are as shown in FIGS. 6 to 9, respectively.

[Table 2]

| Ratio (%) depending on oxidation number of Cu / content (at %) depending on oxidation number of Cu | Cu-O-Al catalyst | | | |
|---|---|---|---|---|
| | Cu/Al wt% ratio = 15/85 | | Cu/Al wt% ratio = 30/70 | |
| | Comp. Ex. 2-2 | Comp. Ex. 2-3 | Ex. 2-3 | Comp. Ex. 2-4 |
| Cu(0), Cu(I) | 53 / 1.2 | 17 / 0.2 | 13 / 0.8 | 8 / 0.1 |
| Cu (II) | 47 / 1.0 | 83 / 0.9 | 87 / 5.8 | 92 / 1.0 |

**Test Example 2-1. Evaluation of hydrogen consumption rate (physical property evaluation 1)**

100 g of a feed solution (HPA:2-EH = 1:4) for preparing neopentyl glycol (NPG) and 3 cc of each of the copper-based composite catalysts of Examples 2-1 to 2-3 and Comparative Examples 2-1 and 2-2 were placed in a Buchi autoclave. After reduction treatment with hydrogen at 180 °C, the hydrogen consumption rate (mL/min·$g_{cat}$) at 30 bar and 115 °C was measured for evaluation.

The hydrogen consumption rate measured in Example 2-1 was set to 100%, and the hydrogen consumption rates of Examples 2-2 and 2-3, and Comparative Examples 2-1 and 2-2 were converted to relative percentage (relative hydrogen consumption rate) as compared to Example 2-1. The evaluation result is shown in Table 3.

[Table 3]

| Catalyst type | Classific ation | Copper content (at%, surface) | Ratio (%) of copper with oxidation numbers of 0 and 1 | Ratio (%) of copper with oxidation number of 2 | Relative speed of hydrogen consumpt ion (unit: %) |
|---|---|---|---|---|---|
| Cu-O-Si catalyst | Ex. 2-1 | 3.7 | 18 | 82 | 100 |
| | Comp. Ex. 2-1 | 8.1 | 7 | 93 | 86 |
| | Ex. 2-2 | 3.8 | 27 | 73 | 102 |
| Cu-O-Al catalyst | Comp. Ex. 2-2 | 2.2 | 53 | 47 | 57 |
| | Ex. 2-3 | 6.6 | 13 | 87 | 91 |

As seen from Table 3, the copper-based composite catalysts (Examples 2-1 to 2-3) according to the present disclosure, which contain copper at a content of 2.5 at% to 7.5 at% on the surface, and contain copper with an oxidation number of 0 and copper with an oxidation number 1 of at a ratio of 10% to 50%, and copper with an oxidation number of 2 at a ratio of 50% to 90%, exhibited a hydrogen consumption rate exceeding 90%. In contrast, when the above content (at%) and ratio (%) were not satisfied, the hydrogen consumption rate was lower than 90%. This confirms that the above content and ratio are significant factors for the reactivity of the copper-based composite catalyst. In particular, the same type of catalysts of the examples and the comparative examples showed more significant difference.

**Test Example 2-2. Evaluation of strength reduction rate (physical property evaluation 2)**

**<Comparative Example 3-1>**

The copper-based composite catalyst of Comparative Example 2-2 (before hydrothermal treatment) and the copper-based composite catalyst of Comparative Example 2-3 (after hydrothermal treatment) were compared.

The strength (unit: N) of the catalysts before and after hydrothermal treatment was evaluated. Specifically, the hydrothermal treatment was performed by putting 100 mL of water ($H_2O$) and the copper-based composite catalyst before hydrothermal treatment in an autoclave and maintaining temperature at 150 °C for 3 hours. The pressure inside the autoclave was set to a high pressure between 10 and 20 bar. The result of evaluating the strength reduction rate is as shown in Table 4. The strength reduction rate was calculated as a percentage (%) of '(strength measured before hydrothermal treatment - strength measured after hydrothermal treatment) / (strength measured before hydrothermal treatment)'.

**<Example 3-1>**

The strength reduction rate was evaluated in the same manner as in Comparative Example 3-1, except that the copper-based composite catalyst of Example 2-3 (before hydrothermal treatment) was compared with the copper-based composite catalyst of Comparative Example 2-4 (after hydrothermal treatment). The result is shown in Table 4.

[Table 4]

| Classificaiton | Before hydrothermal treatment | After hydrothermal treatment | Strength reduction rate (%) |
|---|---|---|---|
| Comparative Example 3-1 | 132 | 5 | 96% |
| Example 3-1 | 62 | 62 | 0% |

As shown in Table 4, the copper-based composite catalyst according to the present disclosure before hydrothermal treatment (Example 2-3), which contains copper at a content of 2.5 at% to 7.5 at% on the surface, and contains copper with an oxidation number of 0 and copper with an oxidation number of 1 at a ratio of 10% to 50%, and copper with an oxidation number of 2 at a ratio of 50% to 90%, exhibited a strength reduction rate of 0% after the hydrothermal treatment (see Example 3-1), whereas the copper-based composite catalyst before the hydrothermal treatment, which does not satisfy the above content (at%) and ratio (%), exhibited a strength reduction rate of 96% (see Comparative Example 3-1).

## Claims

**1.** A copper-based composite catalyst comprising copper at a content of 2.5 at% to 7.5 at% on the surface, wherein

the copper comprises copper with an oxidation number of 0 and copper with an oxidation number of 1 at a ratio of 10% to 50% and copper with an oxidation number of 2 at a ratio of 50% to 90%, and
the content and ratio are determined by the following method 1:
[Method 1]

an XPS spectrum is obtained for the surface of the copper-based composite catalyst, which includes a main peak for copper with an oxidation number of 0, a main peak for copper with an oxidation number of 1, a main peak for copper with an oxidation number of 2, and satellite peaks for copper, and
the total content in at% of copper and the ratio in % of copper with oxidation numbers of 0 and 1 and copper with an oxidation number of 2 are determined using the area of the XPS spectrum.

**2.** The copper-based composite catalyst according to claim 1, wherein

the ratio in % of copper with oxidation numbers of 0 and 1 is calculated from Equation 1, and
the ratio in % of copper with an oxidation number of 2 is calculated from Equation 2:

[Equation 1]

$$\%\,(\mathrm{Cu}(0)+\mathrm{Cu}(\mathrm{I}))=\frac{(\mathrm{A1}+\mathrm{A2})-(\mathrm{As}/\mathrm{Bs})\mathrm{B}}{(\mathrm{A1}+\mathrm{A2})+\mathrm{B}}\times 100(\%)$$

[Equation 2]

$$\%\,\mathrm{Cu}(\mathrm{II})=\frac{\mathrm{B}(1+(\mathrm{As}/\mathrm{Bs}))}{(\mathrm{A1}+\mathrm{A2})+\mathrm{B}}\times 100(\%)$$

wherein

Cu(0), Cu(I), and Cu(II) represent copper with an oxidation number of 0, copper with an oxidation number of 1, and copper with an oxidation number of 2, respectively, and satisfy the following relationship:

$$\%\,(\mathrm{Cu(0)} + \mathrm{Cu(I)}) + \ \%\,\mathrm{Cu(II)} = 100(\%)$$

,

A1 is the sum of the total area of the Cu(0) main peak and the total area of the Cu(I) main peak calculated from the XPS spectrum,
A2 is the total area of the Cu(II) main peak calculated from the XPS spectrum,
B is the total area of satellite peaks for Cu(II) calculated from the XPS spectrum, and
As/Bs is the area ratio of main peak/satellite peaks for Cu $2p_{3/2}$ of Cu (II) .

**3.** The copper-based composite catalyst according to claim 1, wherein the surface means a region having a depth of 0 nm or larger and 10 nm or smaller in a center direction from the interface of the copper-based composite catalyst in contact with the atmosphere.

**4.** The copper-based composite catalyst according to claim 1, wherein the copper-based composite catalyst comprises at least one of a copper oxide and a copper structure.

**5.** The copper-based composite catalyst according to claim 4, which further comprises a support.

**6.** A method for discriminating the oxidation number of a copper-based composite catalyst, comprising:

a step of obtaining an XPS spectrum for the surface of a copper-based composite catalyst, which includes a main peak for copper with an oxidation number of 0, a main peak for copper with an oxidation number of 1, a main peak for copper with an oxidation number of 2, and satellite peaks for copper; and
a step of calculating the total content in at% of copper and the ratio in % of copper with oxidation numbers of 0 and 1 and copper with an oxidation number of 2 using the area of the XPS spectrum.

**7.** The method for discriminating the oxidation number of a copper-based composite catalyst according to claim 6, wherein

the ratio in % of copper with oxidation numbers of 0 and 1 is calculated from Equation 1, and
the ratio in % of copper with an oxidation number of 2 is calculated from Equation 2:

[Equation 1]

$$\%\,(\mathrm{Cu(0)} + \mathrm{Cu(I)}) = \frac{(\mathrm{A1} + \mathrm{A2}) - (\mathrm{As/Bs})\mathrm{B}}{(\mathrm{A1} + \mathrm{A2}) + \mathrm{B}} \times 100(\%)$$

[Equation 2]

$$\%\,\mathrm{Cu(II)} = \frac{\mathrm{B}(1 + (\mathrm{As/Bs}))}{(\mathrm{A1} + \mathrm{A2}) + \mathrm{B}} \times 100(\%)$$

wherein

Cu(0), Cu(I), and Cu(II) represent copper with an oxidation number of 0, copper with an oxidation number of 1, and copper with an oxidation number of 2, respectively, and satisfy the following relationship:

$$\%\,(\mathrm{Cu}(0) + \mathrm{Cu}(\mathrm{I})) + \ \%\,\mathrm{Cu}(\mathrm{II}) = 100(\%)$$

,

A1 is the sum of the total area of the Cu(0) main peak and the total area of the Cu(I) main peak calculated from the XPS spectrum,
A2 is the total area of the Cu(II) main peak calculated from the XPS spectrum,
B is the total area of satellite peaks for Cu(II) calculated from the XPS spectrum, and
As/Bs is the area ratio of main peak/satellite peaks for Cu $2p_{3/2}$ of Cu(II).

**8.** The method for discriminating the oxidation number of a copper-based composite catalyst according to claim 6, wherein the step of obtaining the XPS spectrum for the surface of the copper-based composite catalyst comprises:

a step of obtaining the XPS spectrum of the copper-based composite catalyst by X-ray photoelectron spectroscopy (XPS) under conditions of a vacuum atmosphere, a measurement range of 925 eV to 970 eV, and a pass energy of 50 eV; and
a step of correcting the peaks of the XPS spectrum.

**9.** The method for discriminating the oxidation number of a copper-based composite catalyst according to claim 8, wherein, in the step of correcting the peaks of the XPS spectrum, the C-C binding energy for graphite of the C 1s spectrum is fixed to an energy of a certain size, and the binding energy for the main peak for copper is corrected based on the energy of the certain size.

**10.** The method for discriminating the oxidation number of a copper-based composite catalyst according to claim 6, wherein the step of calculating the total content in at% of copper and the ratio in % of copper with oxidation numbers of 0 and 1 and copper with an oxidation number of 2 using the area of the XPS spectrum comprises:

a step of obtaining the total content of copper on the surface of the copper-based composite catalyst from the XPS spectrum;
a step of obtaining a first area value as the total area for the main peak for copper with an oxidation number of 0 and the main peak for copper with an oxidation number of 1 on the surface of the copper-based composite catalyst from the XPS spectrum;
a step of obtaining a second area value as the total area of the main peak for copper with an oxidation number of 2 on the surface of the copper-based composite catalyst from the XPS spectrum;
a step of obtaining a third area value as the total area of the satellite peaks for copper on the surface of the copper-based composite catalyst from the XPS spectrum; and
a step of obtaining the content in at% of copper with oxidation numbers of 0 and 1 and the content in at% of copper with an oxidation number of 2 from Equations 4 and 5:

[Equation 4]

$$\mathrm{at\%}(\mathrm{Cu}(0) + \mathrm{Cu}(\mathrm{I})) = \mathrm{at\%}(\mathrm{Cu}) \times \frac{\%(\mathrm{Cu}(0) + \mathrm{Cu}(\mathrm{I}))}{100(\%)}$$

[Equation 5]

$$\mathrm{at\%}\ \mathrm{Cu}(\mathrm{II}) = \mathrm{at\%}(\mathrm{Cu}) \times \frac{\%\ \mathrm{Cu}(\mathrm{II})}{100(\%)}$$

wherein

at%(Cu(0) + Cu(I)) is the total content of copper with an oxidation number of 0 and copper with an oxidation number of 1 in the copper-based composite catalyst,

at% Cu(II) is the total content of copper with an oxidation number of 2 in the copper-based composite catalyst,
% (Cu(0) + Cu(I)) is the ratio in % of copper with oxidation numbers of 0 and 1 calculated from Equation 1, and
% Cu (II) is the ratio in % of copper with an oxidation number of 2 calculated from Equation 2.

**11.** The method for discriminating the oxidation number of a copper-based composite catalyst according to claim 10, wherein the step of obtaining the first area value comprises:

a step of finding both a main peak for copper with an oxidation number of 0 and a main peak for copper with an oxidation number of 1 from the XPS spectrum; and
a step of calculating the area values of the main peak for copper with an oxidation number of 0 and the main peak for copper with an oxidation number of 1, and then summing the calculated area values.

**12.** The method for discriminating the oxidation number of a copper-based composite catalyst according to claim 10, wherein the step of obtaining the second area value comprises:

a step of finding all main peaks for copper with an oxidation number of 2 from the XPS spectrum; and
a step of calculating the area value of each main peak for copper with an oxidation number of 2, and then summing the calculated area values.

**13.** The method for discriminating the oxidation number of a copper-based composite catalyst according to claim 10, wherein the step of obtaining the third area value comprises:

a step of finding all satellite peaks for copper from the XPS spectrum; and
a step of calculating the area values of each satellite peak for copper, and then summing the calculated area values.

**14.** The method for discriminating the oxidation number of a copper-based composite catalyst according to claim 7, wherein As/Bs is 1.5 to 2.1.

**15.** The method for discriminating the oxidation number of a copper-based composite catalyst according to claim 6, wherein the satellite peaks for the copper are satellite peaks for copper with an oxidation number of 2.

**16.** The method for discriminating the oxidation number of a copper-based composite catalyst according to claim 6, wherein the copper-based composite catalyst comprises at least one of a copper oxide and a copper structure.

**17.** The method for discriminating the oxidation number of a copper-based composite catalyst according to claim 16, wherein the copper-based composite catalyst further comprises a support.

【FIG. 1】

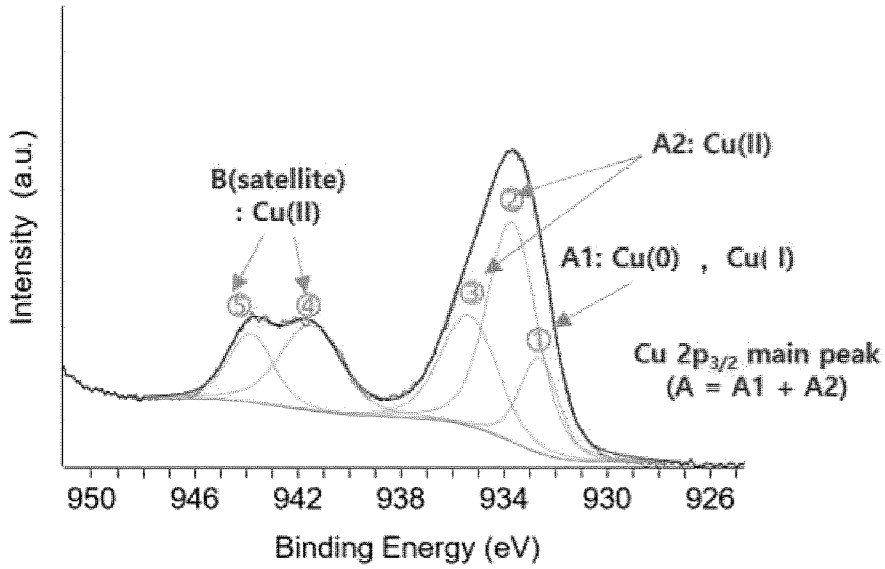
A2: Cu(II)
B(satellite)
: Cu(II)
A1: Cu(0) , Cu( I)
Cu 2p3/2 main peak
(A = A1 + A2)
Intensity (a.u.)
950
946
942
938
934
930
926
Binding Energy (eV)

【FIG. 2】

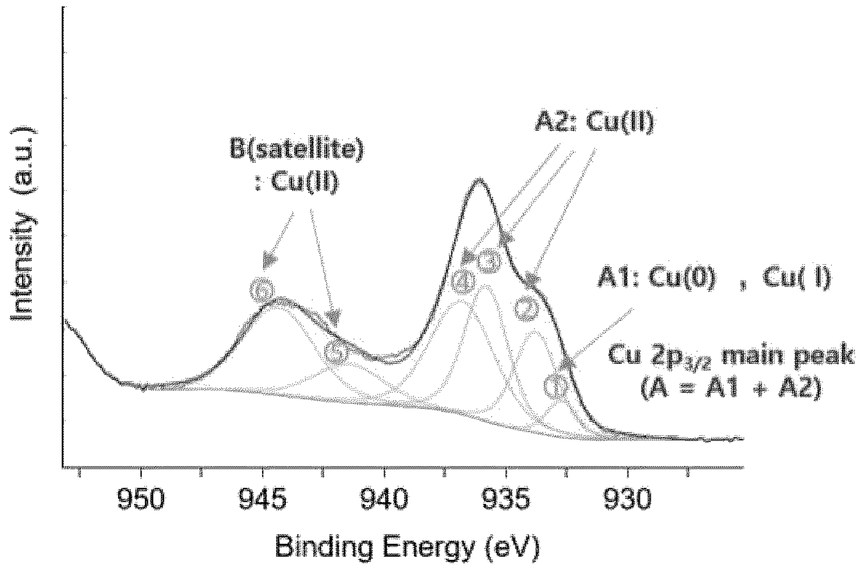
A2: Cu(II)
B(satellite)
: Cu(II)
A1: Cu(0) , Cu( I)
Cu 2p3/2 main peak
(A = A1 + A2)
Intensity (a.u.)
950
945
940
935
930
Binding Energy (eV)

【FIG. 3】

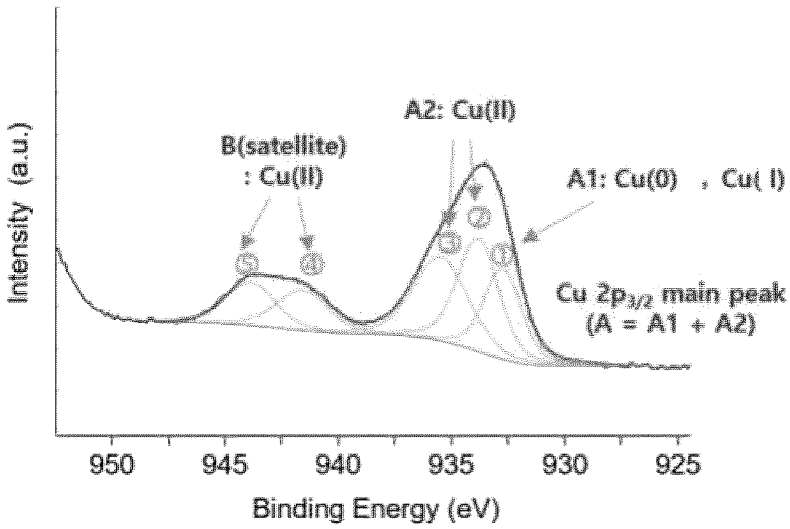
Intensity (a.u.)
B(satellite)
: Cu(II)
A2: Cu(II)
A1: Cu(0) , Cu( I)
Cu 2p3/2 main peak
(A = A1 + A2)
950 945 940 935 930 925
Binding Energy (eV)

【FIG. 4】

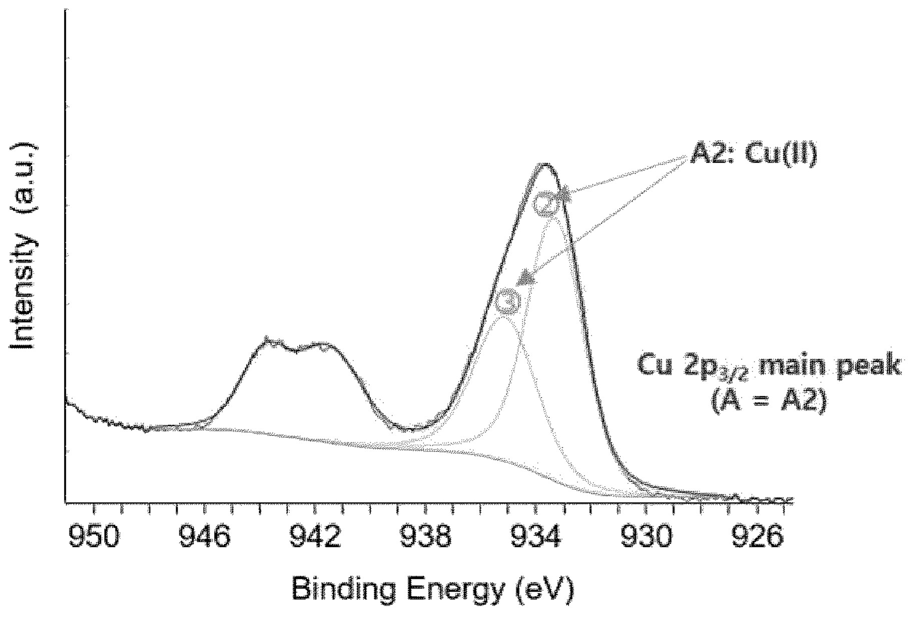
Intensity (a.u.)
A2: Cu(II)
Cu 2p3/2 main peak
(A = A2)
950 946 942 938 934 930 926
Binding Energy (eV)

【FIG. 5】

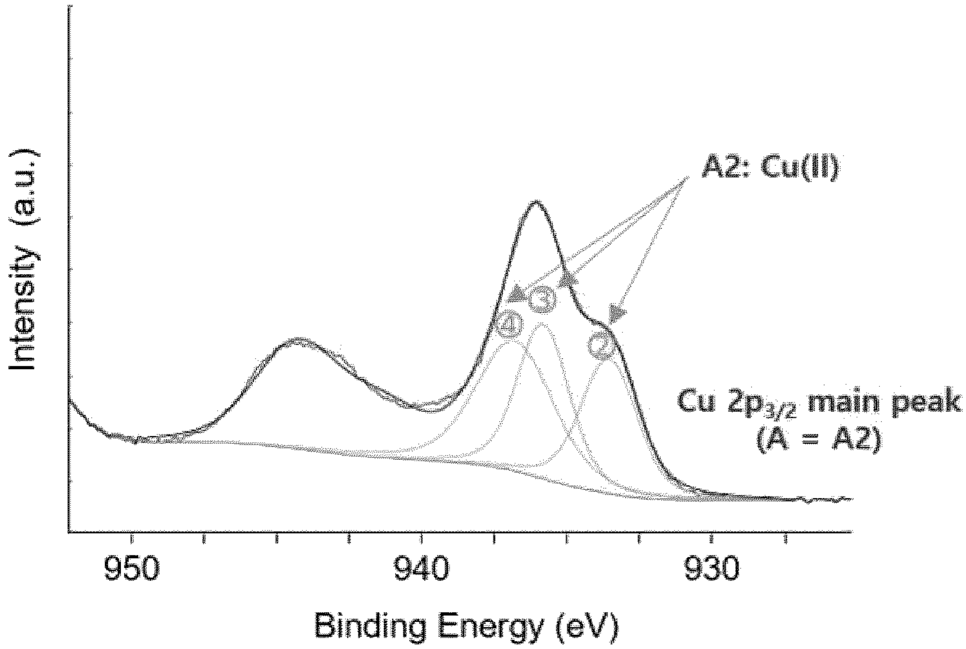
A2: Cu(II)
③
④
②
Cu $2p_{3/2}$ main peak
(A = A2)
Intensity (a.u.)
950
940
930
Binding Energy (eV)

【FIG. 6】

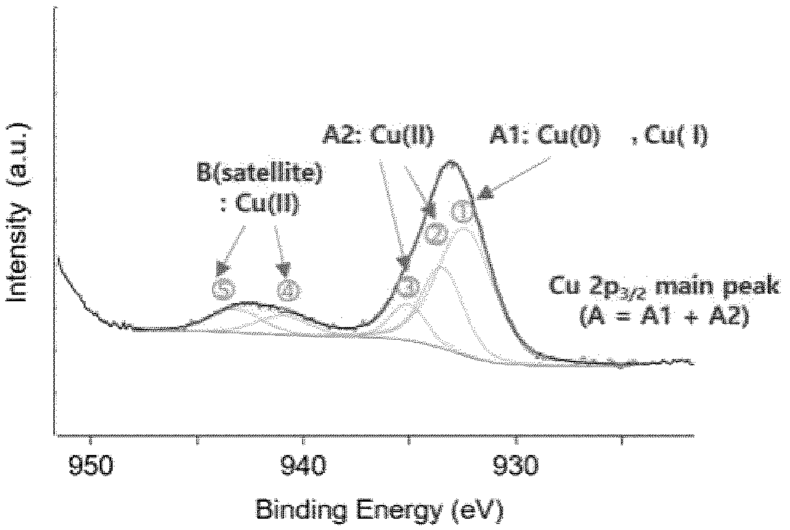
A2: Cu(II)
A1: Cu(0) , Cu( I)
B(satellite)
: Cu(II)
①
②
③
④
⑤
Cu $2p_{3/2}$ main peak
(A = A1 + A2)
Intensity (a.u.)
950
940
930
Binding Energy (eV)

【FIG. 7】

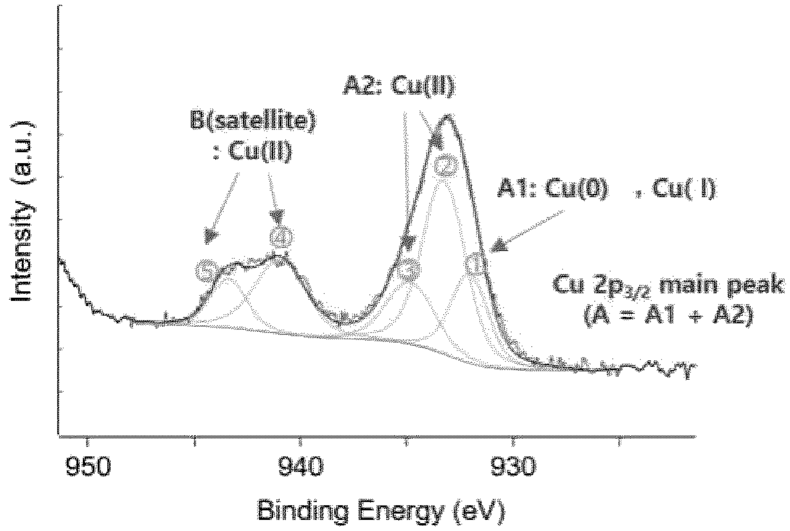
A2: Cu(II)
B(satellite)
: Cu(II)
A1: Cu(0) , Cu( I)
Cu 2p3/2 main peak
(A = A1 + A2)
Intensity (a.u.)
950
940
930
Binding Energy (eV)

【FIG. 8】

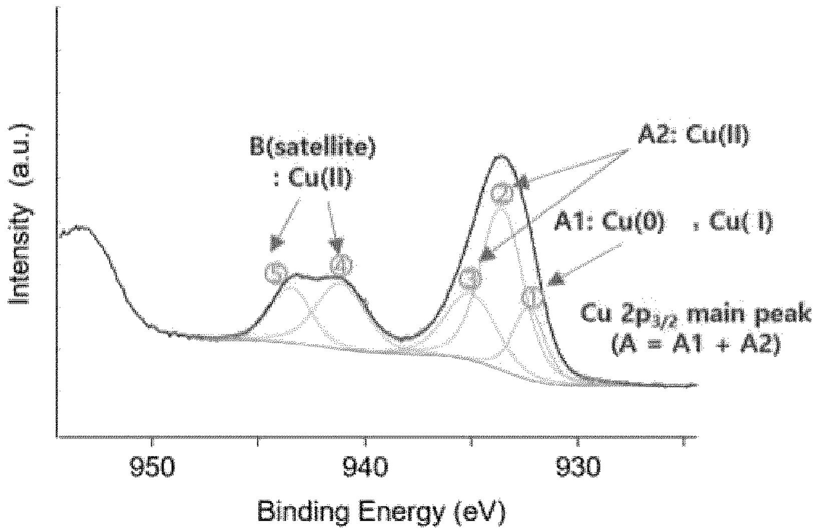
A2: Cu(II)
B(satellite)
: Cu(II)
A1: Cu(0) , Cu( I)
Cu 2p3/2 main peak
(A = A1 + A2)
Intensity (a.u.)
950
940
930
Binding Energy (eV)

【FIG. 9】

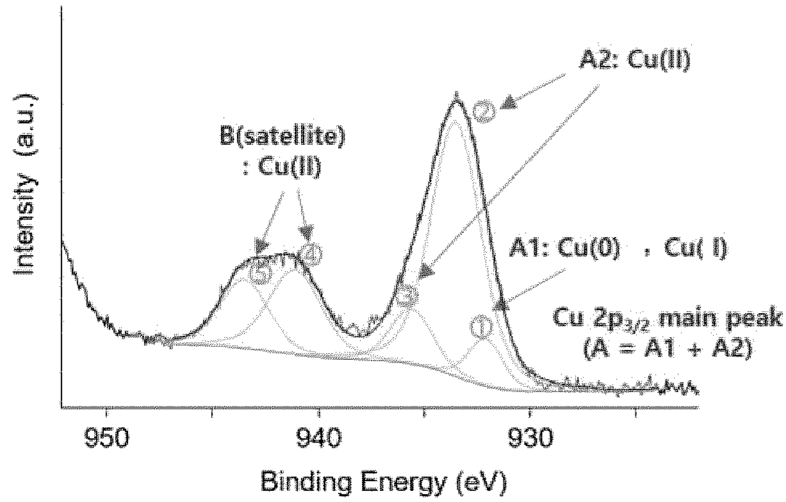
A2: Cu(II)
B(satellite)
: Cu(II)
A1: Cu(0) , Cu( I)
Cu 2p3/2 main peak
(A = A1 + A2)
Intensity (a.u.)
950
940
930
Binding Energy (eV)

**INTERNATIONAL SEARCH REPORT**

International application No.
**PCT/KR2025/001144**

**A. CLASSIFICATION OF SUBJECT MATTER**

**B01J 23/72**(2006.01)i; **B01J 37/00**(2006.01)i; **B01J 37/08**(2006.01)i; **B01J 21/08**(2006.01)i; **B01J 35/50**(2024.01)i; **B01J 37/03**(2006.01)i; **C07C 29/141**(2006.01)i; **C07C 31/20**(2006.01)i

According to International Patent Classification (IPC) or to both national classification and IPC

**B. FIELDS SEARCHED**

Minimum documentation searched (classification system followed by classification symbols)

B01J 23/72(2006.01); B01D 53/94(2006.01); B01J 23/83(2006.01); B22F 1/00(2006.01); B32B 7/023(2019.01); H05K 1/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 구리(copper, Cu), 촉매(catalyst), 산화수(oxidation number), 새틀라이트 피크 (satellite peak)

**C. DOCUMENTS CONSIDERED TO BE RELEVANT**

| Category* | Citation of document, with indication, where appropriate, of the relevant passages | Relevant to claim No. |
|---|---|---|
| A | KR 10-2403998 B1 (MITSUI MINING & SMELTING CO., LTD.) 31 May 2022 (2022-05-31) See claims 1 and 4. | 1-17 |
| A | KR 10-2404788 B1 (DOWA ELECTRONICS MATERIALS CO., LTD.) 02 June 2022 (2022-06-02) See claims 1-7. | 1-17 |
| A | KR 10-2022-0088684 A (NAMICS CORPORATION) 28 June 2022 (2022-06-28) See claims 1-35. | 1-17 |
| A | JP 6741666 B2 (HONDA MOTOR CO., LTD. et al.) 19 August 2020 (2020-08-19) See claims 1-4. | 1-17 |
| A | TORRES-OCHOA, J. A. et al. Peak-fitting of Cu 2p photoemission spectra in Cu0, Cu1+, and Cu2+ oxides: A method for discriminating Cu0 from Cu1+. Applied Surface Science. 2023, vol. 622, document no. 156960, pp. 1-11. See abstract. | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 May 2025** | **02 May 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2025/001144**

| Patent document cited in search report | Publication date (day/month/year) | Patent family member(s) | Publication date (day/month/year) |
|---|---|---|---|
| KR 10-2403998 B1 | 31 May 2022 | CN 110325303 A | 11 October 2019 |
| | | CN 110325303 B | 11 January 2022 |
| | | JP 2020-181482 A1 | 06 February 2020 |
| | | JP 7050756 B2 | 08 April 2022 |
| | | KR 10-2019-0132351 A | 27 November 2019 |
| | | TW 201841702 A | 01 December 2018 |
| | | TW I803486 B | 01 June 2023 |
| | | WO 2018-181482 A1 | 04 October 2018 |
| KR 10-2404788 B1 | 02 June 2022 | CN 107921532 A | 17 April 2018 |
| | | CN 107921532 B | 08 May 2020 |
| | | EP 3345696 A1 | 11 July 2018 |
| | | EP 3345696 B1 | 07 June 2023 |
| | | JP 2017-048461 A | 09 March 2017 |
| | | JP 6807681 B2 | 06 January 2021 |
| | | KR 10-2018-0048980 A | 10 May 2018 |
| | | TW 201715048 A | 01 May 2017 |
| | | TW I689604 B | 01 April 2020 |
| | | US 10773311 B2 | 15 September 2020 |
| | | US 2018-0243831 A1 | 30 August 2018 |
| KR 10-2022-0088684 A | 28 June 2022 | CN 114503789 A | 13 May 2022 |
| | | EP 4050123 A1 | 31 August 2022 |
| | | JP 2021-079952 A1 | 29 April 2021 |
| | | JP 7562153 B2 | 07 October 2024 |
| | | TW 202132622 A | 01 September 2021 |
| | | US 2023-0142375 A1 | 11 May 2023 |
| | | WO 2021-079952 A1 | 29 April 2021 |
| JP 6741666 B2 | 19 August 2020 | CN 109219480 A | 15 January 2019 |
| | | CN 109219480 B | 06 August 2021 |
| | | WO 2017-212944 A1 | 14 December 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020240009953